# EUROPEAN PATENT SPECIFICATION

(11) **EP 3 474 825 B1**
(45) Date of publication and mention of the grant of the patent: **21.07.2021**
(21) Application number: 17772138.8
(22) Date of filing: 21.06.2017
(51) Int. Cl.: A61K 9/50, A61K 31/433

(54) **SUSTAINED RELEASE FORMULATION COMPRISING TIZANIDINE**
RETARD-FORMULIERUNG MIT TIZANIDIN
FORMULATION À LIBÉRATION PROLONGÉE COMPRENANT DE LA TIZANIDINE

(30) Priority: 22.06.2016 TR 201608644
(43) Date of publication of application: 01.05.2019
(73) Proprietor: Santa Farma Ilaç Sanayi A.S., 34382 Istanbul (TR)
(72) Inventor: ILHAN, Doga, Dilovasi, Kocaeli (TR); AKTAS, Tolga, Dilovasi, Kocaeli (TR); MOLLAOGLU, Fatih, Dilovasi, Kocaeli (TR); KANIK, Bayram, Dilovasi, Kocaeli (TR); ÖNER, Levent, Çankaya, Ankara (TR)
(74) Representative: Bulut, Pinar
(86) International application number: PCT/TR2017/050280
(87) International publication number: WO 2017/222488

(56) References cited:
- WO-A2-2008/003093
- US-A1- 2016 030 399
- THRIVENI M ET AL: "Design and evaluation of sustained release multiparticulate system of Tizanidine hydrochloride", INTERNATIONAL JOURNAL OF PHARMACEUTICAL SCIENCES AND RESEARCH - IJPSR, SOCIETY OF PHARMACEUTICAL SCIENCES AND RESEARCH, IN, vol. 4, no. 4, 1 April 2013 (2013-04-01), page 1614, XP009501370, ISSN: 0975-8232, DOI: 10.13040/IJPSR.0975-8232.4(4).1614-25 cited in the application

## Description

### Technical Field of the Invention

The present invention relates to a multiparticulate oral pharmaceutical formulation comprising tizanidine or a pharmaceutically acceptable salt thereof, and the use of the formulation in the manufacture of a medicament for the treatment of painful muscle spasms due to spasticity and musculoskeletal system.

### Background of the Invention

Tizanidine is a central acting muscle relaxant medicine that primarily affects the central nervous system, escpecially the spinal cord. Its antispasmodic activity is due to the agonist effect on central presynaptic alpha 2 - adrenergic receptors. It mainly acts at the level of the spinal cord. It reduces resistance to passive movements, alleviates spasm and muscle contraction, relaxes voluntary muscle strength. It is used to assist in the treatment of spasticity and against painful muscle spasms due to problems in the musculoskeletal system.

The chemical name of tizanidine is 5-chloro-N-(2-imidazolin-2-yl)-2,1,3-benzothiadiazol-4-ylamine and its chemical structure is as shown in Formula 1.

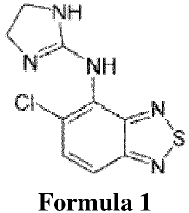

Tizanidine is available as immediate-release tablets (2 mg, 4 mg) and modified release micropellet capsules (6 mg and 12 mg) with SIRALUD® commercial name of Novartis Company; available as immediate release capsules (2, 4, 6 mg) and tablets (2, 4 mg) with ZANAFLEX® commercial name of Acorda (US) and Cephalon (UK).

The SIRDALUD® MR (modified release) micropellet capsule contains 6.864 mg of Tizanidine hydrochloride (Tizanidine HC1) equivalent to 6 mg of Tizanidine as the active ingredient. The initial dose recommended in the treatment of spasticity due to neurological disorders is 6 mg once daily; the daily dose may be increased by 1 capsule of 6 mg every half-week or every week, if necessary. The usual dose ranges from 6 to 24 mg per day. According to clinical experiences, once a day, a total of 12 mg doses given as two 6 mg capsules or one 12 mg capsule was the optimum dose for the majority of patients and rarely 24 mg was required.

SIRDALUD® MR micropellet capsule formulation contains sucrose, ethyl cellulose, shellac, talc, corn starch, gelatin, titanium dioxide (E 171), black iron oxide.

ZANAFLEX® (Acorda) immediate release capsule contains Tizanidine HCl as active ingredient and hydroxypropylmethylcellulose (HPMC), silicon dioxide, sugar spheres, titanium dioxide, gelatin and colorants (US 20140341985 A1). The pellets in the ZANAFLEX® capsule are produced by Elan Corporation with SODAS® (Spheroidal Oral Drug Absorption System) multiparticulate drug delivery technology.

In "Design and evaluation of sustained release multiparticulate system of tizanidine hydrochloride" (Thriveni et al., IJPSR, 2013; Vol. 4(4): 1614-1625), the formulation of various sustained release pellets comprising tizanidine hydrochloride is disclosed.

Patent application of Acorda, US20150038539 A1, relates to a method for increasing the absorption of the tizanidine. In the patent, immediate-release and multiparticle forms comprising Tizanidine HC1 are disclosed. These forms contain sugar spheres. In the formulation, the sugar sphere is coated with a solution containing Tizanidine HCl, hydroxypropylmethylcellulose (HPMC), talc, and preferably organic acid (fumaric acid) using a fluid bed system.

In the article (Thriveni et al., IJPSR, 2013; No. 4 (4): 1614-1625), sustained release multiple particulate forms containing Tizanidine HC1 have been disclosed.

In this study, sugar spheres are coated with 3.5% (w/w) active ingredient, 3% (w/w) polyvinylpyrrolidone as binder (PVP K30), talc and sugar. During this coating process an organic solvent (isopropyl alcohol) is used. The coated sugar spheres are coated with the second layer comprising ethyl cellulose N-50 and (1-4 %w/w) hydroxypropylmethylcellulose E5 (HPMC E5) (1-4% w/w) in 1: 1 ratios, in the presence of organic solvent. The fluid bed method is used for coating this second layer. The obtained pellets (F2-F5 samples in the article) are filled into the capsule. The dissolution profile of product obtained is compared to the dissolution profile of ZANAFLEX® capsule. As a result, it was found that the dissolution profiles of F2-F5 samples were not similar when compared to the innovator product. According to this study, the dissolution profile of sample F7 comprising 2% ethyl cellulose N-50 and 2% Eudragit L-100 combination in the second layer were found to be similar to the dissolution profile of the pellets with the innovator product.

Patent application of Acorda, US20160030399 A1, discloses immediate release oral dosage forms of tizanidine containing multiparticles. In these forms, there are Tizanidine or a pharmaceutically acceptable salt thereof in the range of 2 mg to 12 mg and at least one pharmaceutically acceptable excipient. This formulation is coated on a sugar sphere.

Patent application of Evonik Rohm Gmbh, WO2006102965A1, relates to multiparticulate forms containing controlled release pellets having three layers. In the form, there is an inner core comprising a neutral core, polymer, resin or protein; a layer comprising the active ingredient; and an outer layer comprising methacrylate copolymer. In the patent, it is mentioned that the inner layer contains a shellac.

The shellac is a natural product commonly used as a coating agent on tablet cores. However, there are a number of disadvantages. There are color differences after coating with shellac. The reproducibility of studies with shellac is difficult. There is a supply shortage due to the decrease in production in the market. If shellac is in alcohol solution, its structure changes in storage conditions. Shellac tends to polymerize. This feature can prevent the solid pharmaceutical product from being dispersed in the stomach or intestine as desired. One of the most common problems is the drying of the shellac after a certain period of time. Shellac is creating problems in the production and storage of multi-particulate forms.

The organic solvent is used during the production of the multiparticulate forms. However, the risk of organic solvent toxicity is not preferred due to a number of disadvantages such as the risk of explosion during production. In addition, it is not ecologically suitable because it may also cause environmental contamination. On the other hand, the nonuse of organic solvents is also safer for the patient. Since it is problematic that the residual impurity related to the solvent is present on the finished product. This impurity remains are a danger to human health.

In multiparticle dosage forms, there are particles containing one or more active ingredients dispersed in the subunits. These subunits or particles can be pellets or spherical particles. They typically have a layer-like structure or a matrix structure.

A variety of modified release pharmaceutical forms for oral administration are known. The release of the active ingredient must be controlled as a function of the therapeutic purpose and also of the pharmacological properties of the active ingredient. Multiparticulate dosage forms are particularly used in modified release systems. These forms have many advantages over single unit dosage forms. Multiparticulate systems can produce the desired controlled release behavior and are distributed as subunits. Among the forms, oral preparations still have great importance. Multiparticulate drug forms can be applied to capsules (e.g., hard gelatin capsules) or packaged in a sachet or compressed into tablets.

There are many parameters that are important in the production of the multiparticulate dosage form. For the treatment of a high-quality multiparticulate form, selection of suitable excipients, weight of the form, distribution of ingredients, a suitable production process and a good formulation design are important factors.

### Summary of the Invention

The object of the present invention is to provide an oral sustained release pharmaceutical formulation comprising multiparticulate forms comprising tizanidine or a pharmaceutically acceptable salt thereof, as defined in the claims.

The present invention provides a sustained release formulation in the multiparticulate form comprising tizanidine or a pharmaceutically acceptable salt for oral administration, wherein said multiparticulate form comprises;
- sugar sphere coated with active ingredient layer comprising tizanidine or a pharmaceutically acceptable salt thereof and low-viscosity hydroxypropylmethylcellulose having a viscosity no more than 150 mPa·s (150 cP) at 20°C in 2% aqueous solution;
- active ingredient layer coated with release control layer comprising ethyl cellulose and low-viscosity hydroxypropylmethylcellulose having a viscosity no more than 150 mPa·s (150 cP) at 20°C in 2% aqueous solution; wherein
   a- the ratio of ethyl cellulose: low-viscosity hydroxypropylmethylcellulose having a viscosity no more than 150 mPa·s (150 cP) at 20°C in aqueous solution in the release control layer is in the range of 8:1 to 10:1 by weight, and
   b- in the release control layer the amount of ethylcellulose is 10-30 wt% and the amount of low-viscosity hydroxypropylmethylcellulose having a viscosity no more than 150 mPa·s (150 cP) at 20°C in 2% aqueous solution in the release control layer is 0.5-4 wt %, both wt% ranges based on the total weight of the multiparticulate dosage form.

The invention also provides an oral dosage form comprising said multiparticulate formulation, wherein the dosage form is a capsule, sachet, or tablet.

The invention moreover relates to the use of the multiparticulate form in the manufacture of a medicament, as defined in claim 13.

The oral pharmaceutical formulations may be free of organic solvent.

### Detailed Description of the Invention

The present invention relates to a sustained release formulation comprising tizanidine or a pharmaceutically acceptable salt thereof for oral administration.

The formulation of the invention comprises multiparticulate forms.

The term "multiparticulate" is used as a particle having more than one discrete unit, pellet, granule, bead, minitablet or mixtures thereof.

Important factors in the development of the present invention comprising sustained release multiparticulate forms comprising tizanidine or a pharmaceutically acceptable salt thereof; selection of suitable excipients, weight of the form, distribution of ingredients, a suitable production process and a good formulation design.

The present invention is an oral pharmaceutical formulation comprising sustained release multiparticulate forms comprising tizanidine or a pharmaceutically acceptable salt thereof.

The present multiparticulate form comprises a sugar sphere, an active ingredient layer coating the sugar sphere, and a release control layer coating the active ingredient layer.

The present multiparticulate form comprises an active ingredient layer comprising tizanidine or a pharmaceutically acceptable salt thereof, and binder; a release control layer comprising pore former and release modifier.

For determining the release characteristics of the present invention, the choice of binder, the concentration of the binder (% weight/total weight) are critical parameters. The binder is low viscosity hydroxypropylmethylcellulose with a viscosity of 150 m·Pas (150 centipoise (cP)) or below when measured at 20°C in 2% aqueous solution.

In a preferred embodiment, the low-viscosity hydroxypropylmethylcellulose is selected from a group comprising Methocel™ E5 (5 mPa·s (5 cP)),

Methocel™ E5 LV (5 mPa·s (5 cP)), Methocel™ E15 LV (15 mPa·s (15 cP)), Methocel™ E50 LV (50 mPa·s (50 cP)), Methocel™ K100 LV (100 mPa·s) (100 cP)), Methocel™ F50 LV (50 mPa·s (50 cP)), Methocel™ E3 LV, Methocel™ E6, Methocel™ A15, and mixtures thereof.

Due to the variety of chemical and physical properties of the release modifiers, the release modifier selection, the concentration of the release modifier (% weight / total weight) are critical parameters, especially for determining the release characteristics. The release modifier is ethyl cellulose. Ethyl cellulose is preferably used in the form of aqueous dispersion. The aqueous dispersion form contains ethyl cellulose dry weight in the range of 10 to 50% of the total weight of the aqueous dispersion. Preferably, the aqueous dispersion contains from 10% to 40%, more preferably from 15% to 30%, most preferably from 15% to 25% ethyl cellulose dry weight of the total weight of the aqueous dispersion.

The present invention relates to multiparticulate form comprising an sugar sphere coated with an active ingredient layer comprising tizanidine or a pharmaceutically acceptable salt thereof and low-viscosity hydroxypropylmethylcellulose; and the active ingredient layer coated with a release control layer comprising low-viscosity hydroxypropylmethylcellulose and ethyl cellulose.

In the preferred embodiment of the invention, the oral pharmaceutical formulation comprising the sustained release multiparticulate does not contain organic solvent.

The invention preferably comprises tizanidine or equivalent amount of salt in an amount ranging from 2 mg to 24 mg, preferably from 6 mg to 12 mg. Preferably, the pharmaceutically acceptable salt of tizanidine is used. In the preferred embodiment of the invention, tizanidine HCl is used.

In the preferred embodiment of the invention, the low viscosity hydroxypropylmethylcellulose present in the first layer, which is also the active ingredient layer, is present in certain proportions. In this layer, low viscosity hydroxypropylmethylcellulose is preferably present in an amount of 1 to 10% by weight, more preferably 2 to 6% by weight based on the total weight.

The combination of low viscosity hydroxypropylmethylcellulose and ethyl cellulose in the second layer, the release control layer, is present in certain proportions. In this layer, the low viscosity hydroxypropylmethylcellulose is present in an amount of 0.5 wt% to 4 wt%, based on the total weight of the multiparticulate dosage form. In this layer, hydroxypropylmethylcellulose is used for its pore former function.

The ethyl cellulose co-present with the low viscosity hydroxypropylmethylcellulose in the second layer is present in an amount from 10 to 30% by weight, based on the total weight of the multiparticulate dosage form. In this layer, hydroxypropylmethylcellulose is used for its pore former function.

The combination of low viscosity hydroxypropylmethylcellulose and ethyl cellulose in the second layer is present at certain ratios. Thus, an oral pharmaceutical formulation comprising a multiparticulate form which provides sustained release with the desired dissolution profile is achieved.

The weight ratio of ethyl cellulose and low viscosity hydroxypropylmethylcellulose in the release control layer is 8:1 to 10:1.

The multiparticulate form is preferably filled into a soft or hard gelatin capsule, or packaged in a sachet or compressed into tablets.

In a preferred embodiment of the invention, at least one sustained release multiparticulate form is present in a soft or hard gelatin capsule.

The pharmaceutical formulation of the invention is used for the treatment of painful muscle spasms due to problems with spasticity and musculoskeletal system.

In the preferred embodiment of the invention, the multi particulate form is pellet.

In a preferred embodiment of the invention, the pellets are filled in a hard gelatin capsule.

The dissolution profile of the present invention containing said multiparticulate form is determined according to the following procedure:
The dissolution test is carried out at 100 rpm for 24 hours using the USP apparatus I (basket) in each of the following media. As the dissolution medium;
900 ml of 0.1 N HC1, for pH 1.2 medium,
900 ml of acetate buffer for pH 4.5 medium, and
900 ml of phosphate buffer is used for pH 6.8 medium.

The amount of time-dependent released active ingedient was determined by HPLC.

In the present invention, the active ingredient has a pH-independent dissolution profile; an oral dosage form comprising a multiparticulate form in any of the above dissolution media displays a dissolution profile that results in:
- no more than 25% within the first hour,
- at least 40% after 4 hours and
- at least 85% tizanidine released after 12 hours.

According to the present invention, the desired dissolution profile is provided by the different characteristic features of the two layers covering the sugar sphere. More particularly, the desired dissolution profile is provided by means of certain ratios of the release control layer content.

In the preferred embodiment of the invention oral dosage forms are prepared from multiparticulate forms. Oral dosage forms may contain, in addition to the multiparticulate forms, one or more excipients selected from the group consisting of filler, lubricant, antistatic agent, dispersant, surfactant, glidant.

In one embodiment of the invention, said dosage forms comprise a pharmaceutically acceptable filler selected from the group comprising calcium sulfate, dibasic calcium phosphate, lactose, mannitol, microcrystalline cellulose, starch, sucrose and mixtures thereof.

In a preferred embodiment of the invention said dosage forms comprise a pharmaceutically acceptable lubricant selected from the group comprising calcium stearate, glycerin, vegetable oil, magnesium stearate, mineral oil, polyethylene glycol, propylene glycol group and mixtures thereof.

In a preferred embodiment of the invention, said dosage forms comprise a pharmaceutically acceptable antistatic agent selected from the group comprising kaolin, talc, silicon dioxide and mixtures thereof.

In the preferred embodiment of the invention, said dosage forms comprise a pharmaceutically acceptable disintegrant selected from the group comprising alginate, croscarmellose sodium, crospovidone, sodium starch glycollate, pregelatinized starch and mixtures thereof.

In the preferred embodiment of the invention, surfactant is selected from the group comprising polysorbate, sodium lauryl sulfate and mixtures thereof.

In the preferred embodiment of the invention, glidant is selected from the group comprising colloidal silicon dioxide, magnesium stearate, starch, talc and mixtures thereof.

In the present invention, frequently used coating methods known in the pharmaceutical industry can be used to accomplish coatings in the form of multiparticulates. However, the fluidized bed coating system is particularly preferred.

In a preferred embodiment of the invention said multi particulate form comprises:
- 50% to 80% by weight of sugar sphere,
- in the active ingredient layer;
- 3% to 10% by weight of tizanidine hydrochloride
- 2% to 6% by weight of low viscosity HPMC
- in the release control layer;
- 10% to 30% by weight of ethyl cellulose
- 0.5% to 4% of low viscosity HPMC
- 0.1 to 10% by weight of talc

Also disclosed, but not claimed, is a method of preparing capsule dosage form comprising multiparticulate form comprising the steps of:
- Preparation of coating solutions
- Coating of sugar sphere with coating solution containing active ingredient and binder,
- Coating of the active ingredient layer with coating solution containing pore former and release modifier,
- drying of the multiparticulate form after coating,
- filling multi-particulate forms into hard gelatin capsule

The following examples are intended to further illustrate the invention and are not to be construed as limiting the invention.

### Examples

### Example 1 - Multiparticulate Form Composition

The table below (Table 1) contains the formulation information of the oral pharmaceutical forms comprising the multiparticulate. The multiparticulate forms are in pellet form. Examples I, II, and V to VIII are reference examples.

**Table 1. Examples of Multiparticulate Oral Pharmaceutical Form**

| | **Example I (%w/w)** | **Example II (%w/w)** | **Example III (%w/w)** | **Example IV (%w/w)** | **Example V (%w/w)** | **Example VI (%w/w)** | **Example VII (%w/w)** | **Example VIII (%w/w)** |
|---|---|---|---|---|---|---|---|---|
| **Inner Core** | | | | | | | | |
| **Sugar Sphere** | 75.53 | 75.03 | 76.22 | 76.47 | 76.72 | 76.96 | 77.09 | 62.28 |

| **Active Ingredient Layer** | | | | | | | | |
|---|---|---|---|---|---|---|---|---|
| **Tizanidin HCl** | 4.47 | 4.42 | 4.49 | 4.51 | 4.52 | 4.53 | 4.56 | 3.54 |
| **Hydroxypro pyl methyl cellulose (HPMC)** | 4.40 | 4.40 | 4.47 | 4.49 | 4.50 | 4.51 | 4.50 | 3.52 |

| **Release Control Layer** | | | | | | | | |
|---|---|---|---|---|---|---|---|---|
| **Ethyl Cellulose** | 15.20 | 12.58 | 12.78 | 12.82 | 12.87 | 12.91 | 12.93 | - |
| **Hydroxypro pyl methyl cellulose (HPMC)** | - | 3.15 | 1.60 | 1.28 | 0.99 | 0.5 | 0.52 | 1.38 |
| **Talc** | 0.40 | 0.42 | 0.43 | 0.43 | 0.40 | 0.44 | 0.40 | 15.41 |
| **Eudragit NE30D** | - | - | - | - | - | - | - | 13.87 |

The weight ratios of ethylcellulose to HPMC in the release control layer of the multiparticulate oral pharmaceutical formulations of Table 1 are given below (Table 2).

**Table 2. Weight Ratios of Ethyl Cellulose: HPMC in the Release Control Layer**

| | **Example I** | **Example II** | **Example III** | **Example IV** | **Example V** | **Example VI** | **Example VII** | **Example VIII** |
|---|---|---|---|---|---|---|---|---|
| **Ethyl Cellulose :HPMC** | * | 4:1 | 8:1 | 10:1 | 13:1 | 20:1 | 25:1 | ** |

| | | | | | | | | |
|---|---|---|---|---|---|---|---|---|
| * It contains only Ethyl cellulose, not HPMC. ** It is the Eudragit® NE30D to HPMC ratio. The ratio is 10: 1. | | | | | | | | |

Coating of pellets containing tizanidine HCl was carried out according to the formulations given in Table 1. The sugar sphere is coated with the active ingredient layer, and subsequently the release control layer. Methocel™ E5, ethyl cellulose (Surelease®) and Eudragit® NE30D are placed in different concentrations in coating solutions prepared in water presence. Coating and drying processes were performed with Solidlab II fluid bed dryer system.

The coating conditions of the pellets are as follows (Table 3):

**Table 3.** Fluid Bed Coating Parameters

**Table 3. Fluid Bed Coating Parameters**

| | |
|---|---|
| Spray speed | 10 g/min |
| Atomized air pressure | 1.8 bar |
| Input air flow rate | 150 m³/h |
| Inlet ait temperature | 60°C |
| Product temperature | 40°C |

The coated pellets are dried in a fluidized bed at 40°C for about 20 minutes.

During the drying process, the air flow rate is maintained at about 150 m fh. Solidlab II fluid bed dryer system was used for coating and drying processes.

The pellets are filled into a hard gelatin capsule.

### Example 2 - Dissolution Test

Dissolution tests were carried out on the formulations in Example 1 in the light of the following parameters. Each capsule contains 6 mg Tizanidine equivalent to Tizanidine HC1 salt.

**Table 4. Dissolution Test Parameters**

| | |
|---|---|
| Dissolution Media | 0.1 N HCl (24 h) **or** |
| | pH 4.5 acetate buffer (24 h) **or** |
| | pH6.8 phosphate buffer (24 h) |
| Volume | For 0.1 N HCl: 900 mL |
| | For pH 4.5 acetate buffer: 900 mL |
| | For pH:6.8 phosphate buffer : 900 mL |
| Apparatus | Basket |
| Temperature | 37 °C |
| Speed | 100 rpm |
| Analysis Method | HPLC |

### Example 3 -Comparative Results of Dissolution Tests

The dissolution test results below were performed using the pH 6.8 phosphate buffer (24 hours) in Table 4.

Formulation examples (I- VII) were compared against 24-hour drug release in pH 6.8 phosphate buffer. Examples I, II, and V to VII are reference formulations.

**Table 5. Dissolution Profile (Example I- VII)**

| **Time (hour)** | **% Drug Release** | | | | | | |
|---|---|---|---|---|---|---|---|
| | ***Example* I** | ***Example II*** | ***Example III*** | ***Example IV*** | ***Example* V** | ***Example VI*** | ***Example VII*** |
| **1** | 12 | 27 | 17 | 10 | 19 | 17 | 8 |
| **2** | 29 | 43 | 22 | 21 | 24 | 23 | 15 |
| **3** | 41 | 60 | 36 | 46 | 42 | 44 | 23 |
| **4** | 50 | 73 | **57** | **54** | **53** | **59** | 30 |
| **5** | 57 | 86 | 75 | 71 | 61 | 65 | 36 |
| **6** | 60 | 91 | 79 | 76 | 66 | 69 | 43 |
| **7** | 63 | 97 | 93 | 81 | 71 | 73 | 49 |
| **9** | 71 | 99 | 97 | 86 | 77 | 78 | 54 |
| **12** | 79 | 99 | **98** | **89** | **80** | **82** | 68 |
| **13** | 82 | 99 | 99 | 91 | 83 | 84 | 77 |
| **19** | 90 | 100 | 99 | 93 | 88 | 89 | 84 |
| **24** | 94 | 100 | 100 | 93 | 89 | 92 | 92 |

The dissolution test results below were performed using 0.1 N HCl (24 hours), pH 4.5 acetate buffer (24 hours) and pH: 6.8 phosphate buffer (24 hours) in Table 4. Example IV (ethyl cellulose: HPMC to weight ratio 10: 1) was used for the test.

**Table 6. Example IV dissolution test in three pH media**

| **Time (hour)** | **% Drug Release** | | |
|---|---|---|---|
| | ***Example IV*** | | |
| | ***pH: 1.2*** | ***pH: 4.5*** | ***pH: 6.8*** |
| **1** | **6** | **7** | **10** |
| **2** | 12 | 14 | 21 |
| | 15 | | |
| 3 | 27 | 29 | 46 |
| 4 | 40 | 43 | 54 |
| 5 | 55 | 59 | 71 |
| 6 | 61 | 65 | 76 |
| 7 | 69 | 71 | 81 |
| 9 | 75 | 79 | 86 |
| 12 | 88 | 85 | 89 |
| 13 | 90 | 94 | 91 |
| 19 | 95 | 98 | 93 |
| 24 | 97 | 99 | 93 |

The dissolution test results in Table 7 below were obtained in 900 ml pH 6.8 phosphate buffer (24 hours).

Example IV (ethyl cellulose: HPMC to weight ratio 10: 1) and Reference Example VII (Eudragit: HPMC to weight ratio 10: 1) were used for the test.

**Table 7. Comparative Dissolution test results of Example IV and Reference Example VII**

| **Time (hour)** | **% Drug Release** | |
|---|---|---|
| | ***Example IV*** | ***Example VII*** |
| **1** | 10 | 41 |
| **2** | 21 | 82 |
| **3** | 46 | 93 |
| **4** | 54 | 97 |
| **5** | 71 | 98 |
| **6** | 76 | 99 |
| **7** | 81 | 100 |
| **9** | 86 | 100 |
| **12** | 89 | 100 |
| **13** | 91 | 100 |
| **19** | 93 | 100 |
| **24** | 93 | 100 |

It is possible to develop a wide variety of applications around these basic concepts, and the invention, which is as set forth in the claims, can not be limited to the examples disclosed herein.

## Claims

1. A sustained release formulation in the multiparticulate form comprising tizanidine or a pharmaceutically acceptable salt for oral administration, wherein said multiparticulate form comprises;
- sugar sphere coated with active ingredient layer comprising tizanidine or a pharmaceutically acceptable salt thereof and low-viscosity hydroxypropylmethylcellulose having a viscosity no more than 150 mPa·s (150 cP) at 20°C in 2% aqueous solution;
- active ingredient layer coated with release control layer comprising ethyl cellulose and low-viscosity hydroxypropylmethylcellulose having a viscosity no more than 150 mPa·s (150 cP) at 20°C in 2% aqueous solution; wherein
a- the ratio of ethyl cellulose: low-viscosity hydroxypropylmethylcellulose having a viscosity no more than 150 mPa·s (150 cP) at 20°C in 2% aqueous solution in the release control layer is in the range of 8:1 to 10:1 by weight, and
b- in the release control layer the amount of ethylcellulose is 10-30 wt% and the amount of low-viscosity hydroxypropylmethylcellulose having a viscosity no more than 150 mPa·s (150 cP) at 20°C in 2% aqueous solution in the release control layer is 0.5-4 wt%, both wt% ranges based on the total weight of the multiparticulate dosage form.

2. The multiparticulate according to claim 1, wherein the tizanidine salt is tizanidine hydrochloride.

3. The multiparticulate form according to claim 1-2, wherein the hydroxypropylmethylcellulose used in the production of multiparticulate form is one or more of the low viscosity hydroxypropylmethylcellulose having a viscosity of 5 mPa·s, 15 mPa·s, 50 mPa·s, or 100 mPa·s (5 cP, 15 cP, 50 cP, or 100 cP).

4. The multiparticulate according to any one of claims 1-3, wherein the multiparticulate form is selected from a group of particle having more than one discrete unit, pellet, granule, bead, minitablet and mixtures thereof.

5. The multiparticulate according to claim 4, wherein the multiparticulate form is pellet.

6. A multiparticulate form according to any of the preceding claims wherein tizanidine or a pharmaceutically acceptable salt thereof is released in accordance with the following dissolution profile as measured in each of the buffers which are 900 ml of 0.1 N HCl, 900 ml of pH 4.5 acetate buffer and 900 ml of pH 6.8 phosphate buffer, at 100 rpm, according to USP apparatus I (basket),
- no more than 25% within the first hour
- at least 40% after 4 hours
- at least 85% after 12 hours

7. The multiparticulate form according to any one of claims 1 to 6, wherein it comprises the following substances in the amounts specified according to the weight of the multiparticulate form:
- 50% to 80% by weight of sugar sphere,
- in the active ingredient layer:
- 3% to 10% by weight of tizanidine hydrochloride
- 2% to 6% by weight of low viscosity HPMC, having a viscosity no more than 150 mPa·s (150 cP) at 20°C in 2% aqueous solution
- in the release control layer:
- 10% to 30% by weight of ethyl cellulose
- 0.5% to 4% of low viscosity HPMC, having a viscosity no more than 150 mPa·s (150 cP) at 20°C in 2% aqueous solution
- 0.1 to 10% by weight of talc

8. The oral dosage form comprising multiparticulate form according to any one of claims 1-7, wherein the dosage form is a capsule, sachet or tablet.

9. The oral dosage form according to claim 8, wherein it is a capsule.

10. An oral dosage form according to any one of claims 8-9, wherein it comprises one or more excipients selected from the group comprising of filler, lubricant, antistatic agent, dispersant, surfactant, glidant and mixtures thereof.

11. The oral dosage form according to any one of claims 8-10, wherein the amount of Tizanidine or pharmaceutically acceptable salt in the unit dosage form is in the range of 2 mg to 24 mg, preferably 6 mg to 12 mg.

12. The oral dosage form according to any one of claims 8-11, wherein tizanidine or a pharmaceutically acceptable salt thereof is released in accordance with the following dissolution profile as measured in each of the buffers which are 900 ml of 0.1 N HC1, 900 ml of pH 4.5 acetate buffer and 900 ml of pH 6.8 phosphate buffer, at 100 rpm according to USP apparatus I (basket),
- no more than 25% within the first hour
- at least 40% after 4 hours
- at least 85% after 12 hours

13. Use of the multiparticulate form according to any of claims 1-10 in the manufacture of a medicament according to any of claims 8-12 for the treatment of painful muscle spasms due to problems with spasticity and musculoskeletal system.

## Patentansprüche

1. Formulierung mit verzögerter Freisetzung in der multipartikulären Form, umfassend Tizanidin oder ein pharmazeutisch akzeptables Salz zur oralen Verabreichung, wobei die multipartikuläre Form umfasst:
- Zuckerkugel, beschichtet mit einer Wirkstoffschicht, die Tizanidin oder ein pharmazeutisch akzeptables Salz davon und niedrigviskose Hydroxypropylmethylcellulose mit einer Viskosität von nicht mehr als 150 mPa-s (150 cP) bei 20°C in 2 %iger wässriger Lösung enthält;
- Wirkstoffschicht, die mit einer Freisetzungskontrollschicht beschichtet ist, die Ethylcellulose und niedrigviskose Hydroxypropylmethylcellulose mit einer Viskosität von nicht mehr als 150 mPa-s (150 cP) bei 20°C in 2 %iger wässriger Lösung umfasst; wobei
a- das Verhältnis von Ethylcellulose: niedrigviskoser Hydroxypropylmethylcellulose mit einer Viskosität von nicht mehr als 150 mPa-s (150 cP) bei 20°C in 2 %iger wässriger Lösung in der Freisetzungskontrollschicht im Bereich von 8:1 bis 10:1 nach Gewicht liegt, und
b- in der Freisetzungskontrollschicht die Menge an Ethylcellulose 10-30 Gew.-% beträgt und die Menge an niedrigviskoser Hydroxypropylmethylcellulose mit einer Viskosität von nicht mehr als 150 mPa-s (150 cP) bei 20°C in 2%iger wässriger Lösung in der Freisetzungskontrollschicht 0,5-4 Gew.-% beträgt, wobei beide Gew.-%-Bereiche auf das Gesamtgewicht der multipartikulären Dosierungsform bezogen sind.

2. Multipartikulat nach Anspruch 1, wobei das Tizanidin-Salz Tizanidin-Hydrochlorid ist.

3. Multipartikuläre Form nach Anspruch 1-2, wobei die bei der Herstellung der multipartikulären Form verwendete Hydroxypropylmethylcellulose eine oder mehrere der niedrigviskosen Hydroxypropylmethylcellulose mit einer Viskosität von 5 mPa-s, 15 mPa-s, 50 mPa-s oder 100 mPa-s (5 cP, 15 cP, 50 cP oder 100 cP) ist.

4. Multipartikulat nach einem der Ansprüche 1 bis 3, wobei die multipartikuläre Form aus einer Gruppe von Partikeln mit mehr als einer diskreten Einheit, Pellet, Granulat, Perle, Minitablette und Mischungen davon ausgewählt ist.

5. Das Multipartikulat nach Anspruch 4, wobei die multipartikuläre Form ein Pellet ist.

6. Multipartikuläre Form nach einem der vorhergehenden Ansprüche, wobei Tizanidin oder ein pharmazeutisch annehmbares Salz davon gemäß dem folgenden Auflösungsprofil freigesetzt wird, wie es in jedem der Puffer gemessen wird, die 900 ml 0,1 N HCI, 900 ml pH 4,5-Acetatpuffer und 900 ml pH 6,8-Phosphatpuffer sind, bei 100 U/min, gemäß USP-Apparat I (Korb),
- nicht mehr als 25% innerhalb der ersten Stunde
- mindestens 40% nach 4 Stunden
- mindestens 85% nach 12 Stunden

7. Multipartikuläre Form nach einem der Ansprüche 1 bis 6, **dadurch gekennzeichnet, dass** sie die folgenden Substanzen in den angegebenen Mengen, bezogen auf das Gewicht der multipartikulären Form, enthält:
- 50 bis 80 Gew.-% Zuckerkugel,
- in der Wirkstoffschicht:
- 3 Gew.-% bis 10 Gew.-% Tizanidinhydrochlorid
- 2 bis 6 Gew.-% niedrigviskoses HPMC mit einer Viskosität von nicht mehr als 150 mPa-s (150 cP) bei 20°C in 2%iger wässriger Lösung
- in der Freisetzungskontrollschicht:
- 10 bis 30 Gew.-% Ethylcellulose
- 0,5 % bis 4 % niedrigviskoses HPMC mit einer Viskosität von nicht mehr als 150 mPa-s (150 cP) bei 20 °C in 2 %iger wässriger Lösung
- 0,1 bis 10 Gew.-% Talkum

8. Orale Darreichungsform mit multipartikulärer Form nach einem der Ansprüche 1-7, wobei die Darreichungsform eine Kapsel, ein Beutel oder eine Tablette ist.

9. Orale Darreichungsform nach Anspruch 8, **dadurch gekennzeichnet, dass** sie eine Kapsel ist.

10. Orale Dosierungsform nach einem der Ansprüche 8-9, wobei sie einen oder mehrere Hilfsstoffe umfasst, die aus der Gruppe ausgewählt sind, die Füllstoff, Gleitmittel, Antistatikum, Dispergiermittel, Tensid, Gleitmittel und Mischungen davon umfasst.

11. Orale Dosierungsform nach einem der Ansprüche 8-10, wobei die Menge an Tizanidin oder pharmazeutisch verträglichem Salz in der Einheitsdosierungsform im Bereich von 2 mg bis 24 mg, vorzugsweise 6 mg bis 12 mg, liegt.

12. Orale Dosierungsform nach einem der Ansprüche 8 bis 11, wobei Tizanidin oder ein pharmazeutisch verträgliches Salz davon gemäß dem folgenden Auflösungsprofil freigesetzt wird, gemessen in jedem der Puffer, die 900 ml 0,1 N HCl, 900 ml pH 4,5 Acetatpuffer und 900 ml pH 6,8 Phosphatpuffer sind, bei 100 U/min gemäß USP-Apparat I (Korb),
- nicht mehr als 25% innerhalb der ersten Stunde
- mindestens 40% nach 4 Stunden
- mindestens 85% nach 12 Stunden

13. Verwendung der multipartikulären Form nach einem der Ansprüche 1-10 bei der Herstellung eines Arzneimittels nach einem der Ansprüche 8-12 zur Behandlung von schmerzhaften Muskelspasmen aufgrund von Problemen mit Spastizität und dem Bewegungsapparat.

## Revendications

1. Formulation à libération prolongée sous forme multiparticulaire comprenant de la tizanidine ou un sel pharmaceutiquement acceptable pour une administration orale, dans laquelle ladite forme multiparticulaire comprend:
- Sphère de sucre enrobée d'une couche de principe actif comprenant de la tizanidine ou un sel pharmaceutiquement acceptable de celle-ci et de l'hydroxypropylméthylcellulose de faible viscosité ayant une viscosité ne dépassant pas 150 mPa-s (150 cP) à 20°C dans une solution aqueuse à 2 %;
- couche de principe actif enrobée d'une couche de contrôle de libération comprenant de l'éthylcellulose et de l'hydroxypropylméthylcellulose de faible viscosité ayant une viscosité non supérieure à 150 mPa-s (150 cP) à 20°C dans une solution aqueuse à 2 % ; dans laquelle
a- le rapport éthylcellulose : hydroxypropylméthylcellulose de faible viscosité ayant une viscosité non supérieure à 150 mPa-s (150 cP) à 20°C en solution aqueuse à 2 % dans la couche de contrôle de libération est dans la plage de 8:1 à 10:1 en poids, et
b- dans la couche de contrôle de libération, la quantité d'éthylcellulose est de 10 à 30 % en poids et la quantité d'hydroxypropylméthylcellulose de faible viscosité ayant une viscosité ne dépassant pas 150 mPa-s (150 cP) à 20°C dans une solution aqueuse à 2 % dans la couche de contrôle de libération est de 0,5 à 4 % en poids, les deux plages de % en poids étant basées sur le poids total de la forme posologique multiparticulaire.

2. La multiparticule selon la revendication 1, dans lequel le sel de tizanidine est le chlorhydrate de tizanidine.

3. La forme multiparticulaire selon la revendication 1-2, dans laquelle l'hydroxypropyl méthylcellulose utilisée dans la production de la forme multiparticulaire est une ou plusieurs de l'hydroxypropyl méthylcellulose de faible viscosité ayant une viscosité de 5 mPa-s, 15 mPa-s, 50 mPa-s, ou 100 mPa-s (5 cP, 15 cP, 50 cP, ou 100 cP).

4. La multiparticule selon l'une des revendications 1 à 3, dans lequel la forme multiparticulaire est choisie dans le groupe des particules ayant plus d'une unité distinctive, des granulés, des billes, des mini-tablettes et de leurs mélanges.

5. La multiparticule selon la revendication 4, dans lequel la forme multiparticulaire est un granulé.

6. Une forme multiparticulaire selon l'une des revendications précédentes dans laquelle la tizanidine ou un sel pharmaceutiquement acceptable de celle-ci est libérée selon le profil de dissolution suivant tel que mesuré dans chacun des tampons qui sont 900 ml de HCI 0,1 N, 900 ml de tampon acétate pH 4,5 et 900 ml de tampon phosphate pH 6,8, à 100 rpm, selon l'appareil USP I (panier),
- pas plus de 25% dans la première heure
- au moins 40 % après 4 heures
- au moins 85 % après 12 heures

7. La forme multiparticulaire selon l'une des revendications 1 à 6, dans laquelle elle comprend les substances suivantes dans les quantités spécifiées en fonction du poids de la forme multiparticulaire
- 50% à 80% en poids de sphère de sucre,
- dans la couche du principe actif :
- 3 % à 10 % en poids de chlorhydrate de tizanidine
- 2 % à 6 % en poids de HPMC à faible viscosité, ayant une viscosité ne dépassant pas 150 mPa-s (150 cP) à 20°C dans une solution aqueuse à 2 %.
- dans la couche de contrôle de libération :
- 10 % à 30 % en poids d'éthylcellulose
- 0,5 % à 4 % de HPMC de faible viscosité, ayant une viscosité ne dépassant pas 150 mPa-s (150 cP) à 20°C dans une solution aqueuse à 2 %.
- 0,1 à 10 % en poids de talc

8. La forme posologique orale comprenant une forme multiparticulaire selon l'une des revendications 1 à 7, dans laquelle la forme posologique est une capsule, un sachet ou un comprimé.

9. La forme posologique orale selon la revendication 8, dans laquelle il s'agit d'une capsule.

10. Une forme posologique orale selon l'une des revendications 8-9, dans laquelle elle comprend un ou plusieurs excipients choisis dans le groupe comprenant une matière de charge, un lubrifiant, un agent antistatique, un dispersant, un tensioactif, un glissant et leurs combinaisons.

11. La forme posologique orale selon l'une des revendications 8-10, dans laquelle la quantité de Tizanidine ou de sel pharmaceutiquement acceptable dans la forme posologique unitaire est dans la plage de 2 mg à 24 mg, de préférence de 6 mg à 12 mg.

12. La forme posologique orale selon l'une des revendications 8-11, dans laquelle la tizanidine ou un sel pharmaceutiquement acceptable de celle-ci est libérée conformément au profil de dissolution suivant tel que mesuré dans chacun des tampons qui sont 900 ml de HCl 0,1 N, 900 ml de tampon acétate pH 4,5 et 900 ml de tampon phosphate pH 6,8, à 100 rpm selon l'appareil USP I (panier),
- pas plus de 25% dans la première heure
- au moins 40 % après 4 heures
- au moins 85 % après 12 heures

13. Utilisation de la forme multiparticulaire selon l'une des revendications 1-10 dans la fabrication d'un médicament selon l'une des revendications 8-12 pour le traitement des spasmes musculaires douloureux dus à des problèmes de spasticité et de l'appareil locomoteur.
